(19) 

(11) **EP 4 796 521 A1**

(12) **EUROPEAN PATENT APPLICATION**
published in accordance with Art. 153(4) EPC

(43) Date of publication:
**26.08.2026 Bulletin 2026/35**

(21) Application number: **24879517.1**

(22) Date of filing: **25.09.2024**

(51) International Patent Classification (IPC):
***C07C 1/02*** *(2006.01)* ***B01J 23/58*** *(2006.01)*
***C01B 3/04*** *(2026.01)* ***C07B 61/00*** *(2006.01)*
***C07C 1/12*** *(2006.01)* ***C07C 9/04*** *(2006.01)*

(52) Cooperative Patent Classification (CPC):
**B01J 23/58; C01B 3/04; C07B 61/00; C07C 1/02; C07C 1/12; C07C 9/04;** Y02E 60/36

(86) International application number:
**PCT/JP2024/034286**

(87) International publication number:
**WO 2025/084091 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 JP 2023180411**
**13.09.2024 JP 2024159324**

(71) Applicant: **SUMITOMO CHEMICAL COMPANY, LIMITED**
**Tokyo 103-6020 (JP)**

(72) Inventors:
- **YONEMOTO, Tetsuro**
  **Ichihara-shi, Chiba 299-0195 (JP)**
- **SEKI, Kohei**
  **Ichihara-shi, Chiba 299-0195 (JP)**
- **NUMAKURA, Masahiko**
  **Ichihara-shi, Chiba 299-0195 (JP)**

(74) Representative: **Vossius & Partner Patentanwälte Rechtsanwälte mbB**
**Siebertstraße 3**
**81675 München (DE)**

# (54) METHOD FOR PRODUCING METHANE

(57) A method for producing methane according the present disclosure includes: producing methane from a raw material gas containing ammonia and carbon dioxide in the presence of a catalyst containing a carrier and a transition metal.



Processed by Luminess, 75001 PARIS (FR)

## Description

CROSS-REFERENCE TO RELATED APPLICATION

This application claims priority to Japanese Patent Application Nos. 2023-180411 and 2024-159324, the disclosure of which is incorporated herein by reference in its entirety.

FIELD

The present disclosure relates to a method for producing methane.

BACKGROUND

From the viewpoint of reducing greenhouse gas emissions, expectations are growing for hydrogen energy obtained by burning hydrogen. However, it is extremely difficult to handle hydrogen during, for example, transporting and storing. For this reason, in recent years, a method has been proposed in which, for example, ammonia produced overseas and imported is used as a hydrogen energy carrier, and hydrogen is produced by decomposing ammonia in contact with a catalyst. For example, Patent Literature 1 discloses that ammonia can be decomposed with high efficiency and hydrogen can be suitably produced by using a catalyst having a carrier on which a transition metal such as ruthenium having a small particle size is supported.

Conventionally, a technology known as methanation has been known in which carbon dioxide is recovered from an exhaust gas or the like and reacted with hydrogen to synthesize methane, a main component of city gas. Methanation enables capturing carbon dioxide emitted by combustion and making it available for reaction with hydrogen. Therefore, methane obtained by methanation is attracting attention as a carbon-neutral fuel. For example, Patent Literature 2 discloses that the use of a methanation catalyst supporting ruthenium as an active metal exhibits high low-temperature activity in the methanation reaction.

CITATION LIST

Patent Literature

Patent Literature 1: JP 2010-194519 A
Patent Literature 2: WO2022/224993 A

SUMMARY

Technical Problem

The ammonia decomposition reaction is a large endothermic reaction, and requires heat supply from outside to maintain the reaction temperature. On the other hand, the reaction of synthesizing methane from carbon dioxide is an exothermic reaction and requires heat removal from the reactor. In other words, there is room for improvement in thermal efficiency in both reactions.

The present disclosure has been made in view of the above circumstances, and an object of the present disclosure is to provide a method for producing methane capable of producing methane with excellent reaction efficiency.

Solution to Problem

A method for producing methane according to the present disclosure includes producing methane from a raw material gas containing ammonia and carbon dioxide in the presence of a catalyst containing a carrier and a transition metal.

BRIEF DESCRIPTION OF DRAWINGS

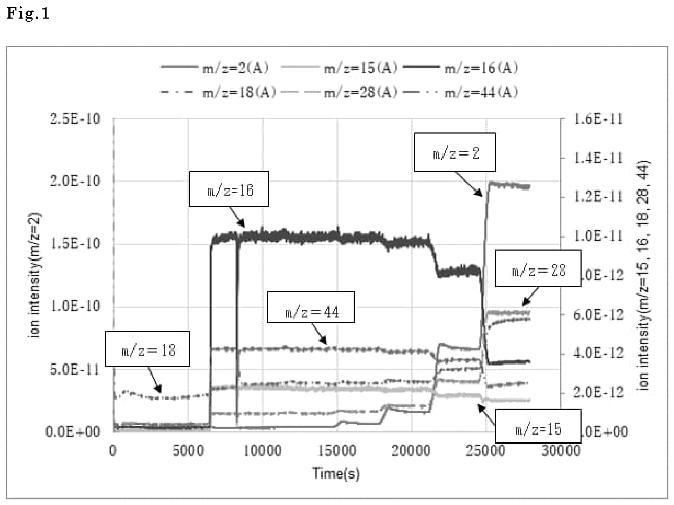

Fig. 1 shows a mass chromatogram of each ion measured by a mass spectrometer in an example of the present disclosure.

DESCRIPTION OF EMBODIMENTS

Although an embodiment of the present disclosure will be hereinafter described, the method for producing methane according to the present disclosure is not limited to the following embodiment, and to the following operation effect. The method for producing methane according to the present disclosure can be modified in various ways without departing from the spirit and scope of the present disclosure.

The method for producing methane according to this embodiment includes: producing methane from a raw material gas containing ammonia and carbon dioxide. In producing methane, a reaction shown in a formula (I) below occurs to produce methane.

$$3.6NH_3+CO_2 \rightarrow 1.8N_2+2H_2O+1.4H_2+CH_4 \quad \text{(I)}$$

More specifically, in producing methane, a reaction shown in a formula (II) below occurs to decompose ammonia, and a reaction shown in a formula (III) below occurs to produce methane. The reaction shown in the formula (II) is an endothermic reaction, and the reaction shown in the formula (III) is an exothermic reaction.

$$NH_3 \rightarrow 1.5H_2+0.5N_2\text{-}45.9\text{kJ/mol} \quad \text{(II)}$$

$$CO_2+4H_2 \rightarrow CH_4+2H_2O+164.7\text{kJ/mol} \quad \text{(III)}$$

The method for producing methane according to this embodiment can produce methane with excellent reaction efficiency by simultaneously proceeding the endothermic reaction shown by the formula (II) and the exothermic reaction shown by the formula (III).

The molar ratio of ammonia to carbon dioxide in the raw material gas is preferably 3.0 or more and 5.4 or less, more preferably 3.0 or more and 5.0 or less, even more preferably 3.2 or more and 4.0 or less. When the molar ratio is 3.0 or more, the method for producing methane according to this embodiment can achieve an exothermic reaction with a reaction heat of 30 kJ/mol or less. This eliminates the need for equipment for heat removal, and reducing capital investment. When the molar ratio is 5.4 or less, the method for producing methane according to this embodiment can achieve an endothermic reaction with a reaction heat of 100 kJ/mol or less. This reduces the amounts of electricity, fuel, steam, and the like used to compensate for the reaction heat, and suppresses the amount of carbon dioxide emissions for the production of these substances.

In one embodiment, the raw material gas further contains hydrogen from the viewpoint of promoting a reaction shown in a formula (III) above. When the raw material gas contains hydrogen, the molar ratio of hydrogen to carbon dioxide in the raw material gas is preferably 0.00001 or more and 10.0 or less, more preferably 0.0001 or more and 5.0 or less. In this embodiment, hydrogen can be produced from the raw material gas simultaneously with the production of methane. Furthermore, in this embodiment, hydrogen contained in the raw material gas can be obtained by recycling a stream containing hydrogen resulting from the production of methane.

Hydrogen contained in the raw material gas can be obtained by recovering hydrogen resulting from the production of methane, and recycling the recovered hydrogen into the raw material gas. Examples of the method for recovering hydrogen resulting from the production of methane include separation of hydrogen by membrane separation and separation of hydrogen by pressure swing adsorption (PSA).

The raw material gas can contain a gas obtained by thermal decomposition of ammonium carbonate. The ammonium carbonate is preferably obtained by bringing an aqueous solution containing ammonia into contact with a gas containing carbon dioxide. Specifically, for example, carbon dioxide gas obtained from a liquefied carbon dioxide gas cylinder is absorbed into 75 mL of a commercially available 28% aqueous ammonia solution at a flow meter value of 100 mL/min for 2 hours. The pH of the solution after 2 hours is 8 to 9. When the solution is left to stand in a refrigerator (4°C) for 24 hours, a crystallized substance is confirmed, and 5.2 g of solid ammonium carbonate can be obtained. The gas containing carbon dioxide can be air or an exhaust gas from a power plant, a factory, a steel mill, a chemical plant, a waste treatment facility, or a biomass facility.

The reaction temperature in the production of methane is preferably 300°C or higher and 550°C or lower, more preferably 350°C or higher and 550°C or lower, even more preferably 400°C or higher and 550°C or lower, from the viewpoint of producing methane with excellent reaction efficiency.

The reaction pressure in the production of methane is preferably 0 MPa-G or more and 0.9 MPa-G or less, more preferably 0.2 MPa-G or more and 0.9 MPa-G or less, even more preferably 0.4 MPa-G or more and 0.9 MPa-G or less, from the viewpoint of producing methane with excellent reaction efficiency.

In one embodiment, the producing of methane is carried out using a reactor. The reactor is preferably at least one selected from the group consisting of an external heat exchange type fixed bed reactor, an adiabatic fixed bed reactor, a fluidized bed reactor, a simulated moving bed reactor, a riser type fluidized bed reactor, and a radial flow type fixed bed

reactor, and is more preferably an adiabatic fixed bed reactor. The reactor can be used alone or in combination of two or more types.

The method for producing methane according to this embodiment is carried out in the presence of a catalyst containing a carrier and a transition metal.

The carrier supports the transition metal. The carrier is preferably at least one carrier selected from the group consisting of titania ($TiO_2$), alumina ($Al_2O_3$), silicon carbide (SiC), carbon (C), carbon nitride ($C_3N_4$), zirconia ($ZrO_2$), silica ($SiO_2$), cerium oxide ($CeO_2$), and niobium oxide (NbO, $Nb_2O_5$), more preferably at least one carrier selected from the group consisting of titania, alumina, silicon carbide, carbon, and cerium oxide, and even more preferably a carbon carrier. The carrier can be used alone or in combination of two or more types.

Examples of the carbon carrier include activated carbon, carbon black, graphene, graphite, acetylene black, mesoporous carbon, carbon nanotubes, carbon nanofibers, and carbon nanohorns. The carbon carrier can contain sulfur as an impurity. The content of sulfur contained in the carbon carrier is preferably 1.8% by mass or less, more preferably 1.0% by mass or less, so as not to impair the effects of the present disclosure.

As the carbon carrier, a commercially available product can be used. Examples of the commercially available product include mesoC+™ manufactured by SICAT SARL and GRANULAR SHIRASAGI (registered trademark) WH2C manufactured by Osaka Gas Chemicals Co., Ltd.

From the viewpoint of producing methane with excellent reaction efficiency, the carrier preferably has pores with an average pore diameter of 2 nm or more and 100 nm or less, more preferably has pores with an average pore diameter of 3 nm or more and 50 nm or less, even more preferably has pores with an average pore diameter of 3.5 nm or more and 15 nm or less.

From the viewpoint of producing methane with excellent reaction efficiency, the specific surface area of the carrier is preferably 5 $m^2/g$ or more and 2000 $m^2/g$ or less, more preferably 10 $m^2/g$ or more and 1500 $m^2/g$ or less.

The average pore diameter (D, unit: nm) and specific surface area (A, unit: $m^2/g$) of the carrier can be determined by vacuum degassing the carrier at 120°C for 8 hours, then measuring the nitrogen adsorption-desorption isotherm at 77K using BELSORP-Max manufactured by Microtrac-Bell Corp, and then using the BET multipoint method and a formula (1) below:

$$D = 4V_P/(A \times 1000) \qquad (1)$$

Here, $V_P$ in the formula (1) represents the total pore volume ($cm^3/g$) up to a relative pressure (P/P0) of 0.99, and can be calculated using a formula (2) below:

$$V_P = (V/22414) \times M_g/\rho_g \qquad (2)$$

In the formula (2), V represents the amount of nitrogen adsorbed ($cm^3/g$) at a relative pressure (P/P0) of 0.99, $M_g$ represents the molecular weight of nitrogen (28.013 g/mol), and $\rho_g$ represents the liquid density of nitrogen (0.808 $g/cm^3$).

From the viewpoint of producing methane with excellent reaction efficiency, the content of the carrier is preferably 70% by mass or more and 99% by mass or less, more preferably 80% by mass or more and 98% by mass or less, based on the entire catalyst.

The shape and size of the carrier are not particularly limited and can be appropriately selected depending on the type, size, operating conditions, or the like of the reactor. Examples of the shape of the carrier include granular, spherical, cylindrical, trilobe, tetralobe, ring, and honeycomb shapes. Regarding the size of the carrier, for example, when the carrier is granular or spherical shape, the average particle size can be 0.5 mm or more and 10 mm or less. Regarding the size of the carrier, for example, when the carrier is cylindrical, trilobe, tetralobe, or ring shape, the average diameter of the cross section or the average length of one side can be 0.5 mm or more and 10 mm or less, and the average length can be 1.0 mm or more and 20 mm or less.

Examples of the transition metal include ruthenium, rhodium, iron, cobalt, and nickel. Among these, the transition metal is preferably ruthenium or nickel, more preferably ruthenium, from the viewpoint of producing methane with excellent reaction efficiency. One type of the transition metal can be used alone, or two or more types can be used in combination.

The transition metal can be derived from salts such as nitrates, carbonates, chlorides, or the like. In one embodiment, ruthenium can be derived from at least one ruthenium compound selected from the group consisting of ruthenium nitrosyl nitrate, ruthenium chloride, ruthenium carbonyl, potassium ruthenate, and ammonium ruthenium chloride. Among these, ruthenium is preferably derived from ruthenium chloride and ruthenium nitrosyl nitrate, and more preferably derived from ruthenium nitrosyl nitrate.

The transition metal can be contained in a catalyst as an elementary substance, or can be contained in a catalyst as an oxide, nitride, phosphide, carbide, or the like. When the transition metal is contained in the catalyst as an oxide, examples of the oxide include ruthenium oxide, iron oxide, cobalt oxide, and nickel oxide.

From the viewpoint of producing methane with excellent reaction efficiency, the content of the transition metal is preferably 1 mass % or more and 30 mass % or less, and more preferably 2 mass % or more and 20 mass % or less, based on the total mass of the catalyst.

From the viewpoint of promoting the reaction, the catalyst can further contain at least one compound selected from the group consisting of an alkali metal compound and an alkaline earth metal compound. Examples of the alkali metal compound and the alkaline earth metal compound include sodium oxide, potassium oxide, cesium oxide, magnesium oxide, calcium oxide, strontium oxide, and barium oxide. These compounds can be used alone or in combination of two or more.

In one embodiment, when the catalyst contains ruthenium oxide and barium oxide, the molar ratio of barium to ruthenium is preferably 0.1 or more and 3 or less, more preferably 0.2 or more and 2 or less.

The catalyst can be prepared by a general method for supporting a transition metal on a carrier. For example, an impregnation method can be used in which a carrier is impregnated with a solution containing the transition metal, followed by drying and calcination. More specifically, the catalyst can be prepared by the following method, for example.

(Preparation of catalyst precursor)

First, a carrier is impregnated with a solution containing a transition metal, which is a catalyst raw material, by an incipient wetness method, followed by drying. The obtained solid is then heated using an electric tubular furnace under the flow of nitrogen, so that a catalyst precursor with a transition metal supported thereon can be obtained. Further, the catalyst precursor is impregnated with a solution containing at least one compound selected from the group consisting of an alkali metal compound and an alkaline earth metal compound, followed by drying. The obtained solid is then heated using an electric tubular furnace under the flow of nitrogen, so that a catalyst precursor containing at least one compound selected from the group consisting of an alkali metal compound and an alkaline earth metal compound in addition to the transition metal can be obtained.

(Preparation of catalyst)

The catalyst can be obtained by heating the obtained catalyst precursor using an electric tubular furnace under the flow of a gas containing hydrogen.

The catalyst thus obtained can be molded into, for example, a spherical, cylindrical or other molded body having an average particle size of 0.5 mm or more and 20 mm or less. When the molded body is spherical, the average particle size means its average diameter, and when the molded body is cylindrical, the average particle size means its average diameter and average length.

The present disclosure includes the following aspects.

[1] A method for producing methane, including: producing methane from a raw material gas containing ammonia and carbon dioxide in the presence of a catalyst containing a carrier and a transition metal.

[2] The method for producing methane according to [1], wherein the molar ratio of ammonia to carbon dioxide in the raw material gas is 3.0 or more and 5.4 or less.

[3] The method for producing methane according to [1] or [2], wherein the raw material gas further contains hydrogen.

[4] The method for producing methane according to [2], wherein hydrogen is produced from the raw material gas simultaneously with the production of methane.

[5] The method for producing methane according to [3], wherein hydrogen contained in the raw material gas is obtained by recovering hydrogen resulting from the production of methane, and recycling the recovered hydrogen into the raw material gas.

[6] The method for producing methane according to [4], wherein hydrogen contained in the raw material gas is obtained by recycling a stream containing hydrogen resulting from the production of methane.

[7] The method for producing methane according to any one of [1] to [6], wherein a reaction temperature in the production of methane is 300°C or higher and 550°C or lower.

[8] The method for producing methane according to any one of [1] to [7], wherein a reaction pressure in the production of methane is 0 MPa-G or more and 0.9 MPa-G or less.

[9] The method for producing methane according to any one of [1] to [8], wherein the carrier is at least one carrier selected from the group consisting of titania, alumina, silicon carbide, carbon, zirconia, silica, cerium oxide, and niobium oxide.

[10] The method for producing methane according to [9], wherein the carrier has pores with an average pore diameter of 2 nm or more and 100 nm or less.

[11] The method for producing methane according to [2], wherein the transition metal is ruthenium.

[12] The method for producing methane according to [3], wherein the transition metal is ruthenium.

[13] The method for producing methane according to [9], wherein the transition metal is ruthenium.

[14] The method for producing methane according to any one of [1] to [13], wherein the production of methane is carried out using a reactor, and the reactor is at least one selected from the group consisting of an external heat exchange type fixed bed reactor, an adiabatic fixed bed reactor, a fluidized bed reactor, a simulated moving bed reactor, a riser type fluidized bed reactor, and a radial flow type fixed bed reactor.

[15] The method for producing methane according to any one of [1] to [14], wherein the raw material gas contains a gas obtained by thermal decomposition of ammonium carbonate.

[16] The method for producing methane according to [15], wherein the ammonium carbonate is obtained by bringing an aqueous solution containing ammonia into contact with a gas containing carbon dioxide.

[17] The method for producing methane according to [16], wherein the gas containing carbon dioxide is air.

[18] The method for producing methane according to [16], wherein the gas containing carbon dioxide is an exhaust gas from a power plant, a factory, a steel mill, a chemical plant, a waste treatment facility, or a biomass facility.

[Examples]

The present disclosure will be described in more detail below using examples and comparative examples, but the present disclosure is not limited to the following examples. In the following description, "%" representing an amount is based on mass unless otherwise specified. Further, the operations described below were performed under conditions of room temperature and normal pressure unless otherwise specified.

[Production Example 1]

<Preparation of catalyst precursor>

A carbon (C) carrier i (mesoC+™ manufactured by SICAT SARL, trilobal pellet shape with a cross section of 1.6 mm and a length of 2 mm, specific surface area (A): 293 $m^2/g$, average pore diameter (D): 5.0 nm) was used. A specific surface area (A) and an average pore diameter (D) of the carrier i were determined by vacuum degassing the carrier i at 120°C for 8 hours, followed by nitrogen adsorption and desorption isotherm measurement at 77K using BELSORP-Max manufactured by Microtrac-Bell Co., Ltd., using the BET multipoint method and a formula (1) below:

$$D = 4V_P/(A \times 1000) \qquad (1)$$

Here, $V_P$ in the formula (1) represents the total pore volume ($cm^3/g$) up to a relative pressure ($P/P_0$) of 0.99, and was calculated using a formula (2) below:

$$V_P = (V/22414) \times M_g/\rho_g \qquad (2)$$

In the formula (2), V represents the amount of nitrogen adsorbed ($cm^3/g$) at a relative pressure ($P/P_0$) of 0.99, $M_g$ represents the molecular weight of nitrogen (28.013 g/mol), and $\rho_g$ represents the liquid density of nitrogen (0.808 $g/cm^3$).

First, 5.2 g of deionized water was added to 2.6 g of ruthenium nitrosyl nitrate in nitric acid aqueous solution (manufactured by Furuya Metal Co., Ltd., $Ru(NO)(NO_3)$, Ru content: 19% by mass) and mixed thoroughly. The obtained solution was impregnated into 10 g of the carrier i by the incipient wetness method, and then air-dried overnight in an air atmosphere at room temperature (approximately 25°C). The obtained solid was packed into a quartz glass tube with an inner diameter of 24 mm equipped with a sheath tube for measuring the inner temperature, and then the tube was heated to an inner temperature of 400°C for 1 hour using an electric tubular furnace under the flow of nitrogen of 100 $cm^3$ (0°C, 0.1013 MPa (absolute))/min, and then maintained at the same temperature for 3 hours, thereby obtaining 10 g of a catalyst precursor i-1 (6.6 wt% $RuO_2$/C) (the loadings of BaO and $RuO_2$ are expressed as parts by mass per 100 parts by mass of support i).

1.9 g of barium nitrate ($Ba(NO_3)_2$, manufactured by Fujifilm Wako Pure Chemical Industries, Ltd.) was added to 10 g of deionized water heated to 80°C and dissolved therein. 10 g of the catalyst precursor i-1 was added to the obtained solution heated to 80°C, and the catalyst precursor i-1 was impregnated with a barium nitrate aqueous solution. Subsequently, the pressure was reduced to 9 to 20 mmHg at 80°C, and the mixture was dried under reduced pressure for 1 hour. The obtained solid was packed into a quartz glass tube with an inner diameter of 24 mm equipped with a sheath tube for measuring the inner temperature, and then the tube was heated to an inner temperature of 500°C for 1 hour using an electric tubular furnace under the flow of nitrogen of 100 $cm^3$ (0°C, 0.1013 MPa (absolute))/min, and then maintained at the same temperature for 1 hour to obtain a catalyst precursor i-2 (11 wt% BaO/6.6 wt% $RuO_2$/C) (the loadings of BaO and $RuO_2$ are expressed as parts by mass per 100 parts by mass of support i). The molar ratio of barium to ruthenium was 1.5.

<Preparation of catalyst>

0.24 g of the catalyst precursor i-2 was packed into a quartz glass tube with an inner diameter of 8 mm equipped with a sheath tube with an outer diameter of 4 mm for measuring the inner temperature, and then the tube was heated to an inner temperature of 400°C using an electric tubular furnace under the flow of hydrogen at 5 $cm^3$ (0°C, 0.1013 MPa (absolute))/min and helium at 95 $cm^3$ (0°C, 0.1013 MPa (absolute))/min, and then maintained at the same temperature for 1 hour to obtain the catalyst i (11 wt% BaO/5.0 wt% Ru/C) (the loadings of BaO and $RuO_2$ are expressed as parts by mass per 100 parts by mass of support i). The catalyst i was cooled to 158°C under the flow of helium of 100 $cm^3$ (0°C, 0.1013 MPa (absolute))/min and used as it was in the methane production reaction.

<Methane production reaction>

(Gas analysis using a mass spectrometer)

Using a gas flow meter, helium gas was flowed at 100 $cm^3$ (0°C, 0.1013 MPa (absolute))/min. Helium gas was introduced into an in-line mass spectrometer (BELMASS manufactured by Microtrac-Bell Corp.), and the ion intensities of m/z=2, 15, 16, 18, 28, and 44 were measured in real time. Each ion intensity 108 minutes after the start of flow was recorded as the baseline intensity.

(Preparation of a raw material gas and gas analysis using a mass spectrometer)

Using a gas flow meter, ammonia gas was flowed at 3.2 $cm^3$ (0°C, 0.1013 MPa (absolute))/min, carbon dioxide gas at 0.90 $cm^3$ (0°C, 0.1013 MPa (absolute))/min, and helium gas at 96 $cm^3$ (0°C, 0.1013 MPa (absolute))/min (total gas flow rate was 100 $cm^3$ (0°C, 0.1013 MPa (absolute))/min). The molar ratio of ammonia to carbon dioxide in the raw material gas was 3.6. The raw material gas was introduced into an in-line mass spectrometer (BELMASS, manufactured by Microtrac-Bell Corp), and the ion intensities of m/z=2, 15, 16, 18, 28, and 44 were measured in real time. Each ion intensity 30 minutes after the start of the flow of the raw material gas was recorded as the raw material gas intensity.

(Methane production reaction 1)

The catalyst i packed in the quartz glass tube was kept at an internal temperature of 158°C, using an electric tubular furnace, and the raw material gas was introduced into the quartz glass tube to carry out methane production reaction 1. The linear velocity of the gas calculated from the flow rate of the raw material gas and the cross-sectional area based on the empty column of the catalyst packed bed was 4.4 cm/sec at 0°C., and 0.1013 MPa (absolute). The post-reaction gas discharged from the quartz glass tube was introduced into an in-line mass spectrometer (BELMASS manufactured by Microtrac-Bell Corp.), and the ion intensities of m/z=2, 15, 16, 18, 28, and 44 were measured in real time. The ion intensity after 46 minutes is shown in Table 1 as "Reaction 1 (158°C)" intensity.

(Methane production reaction 2)

Following the methane production reaction 1, the temperature of the electric tubular furnace was increased at a rate of 5°C/min, and the catalyst i was maintained at an internal temperature of 209°C to carry out the methane production reaction 2. The ion intensity after 58 minutes is shown in Table 1 as "Reaction 2 (209°C)" intensity.

(Methane production reaction 3)

Following the methane production reaction 2, the temperature of the electric tubular furnace was increased at a rate of 5°C/min, and the catalyst i was maintained at an internal temperature of 260°C to carry out the methane production reaction 3. The ion intensity after 52 minutes is shown in Table 1 as "Reaction 3 (260°C)" intensity.

(Methane production reaction 4)

Following the methane production reaction 3, the temperature of the electric tubular furnace was increased at a rate of 5°C/min, and the catalyst i was maintained at an internal temperature of 311°C to carry out the methane production reaction 4. The ion intensity after 57 minutes is shown in Table 1 as "Reaction 4 (311°C)" intensity.

(Methane production reaction 5)

Following the methane production reaction 4, the temperature of the electric tubular furnace was increased at a rate of 5°C/min, and the catalyst i was maintained at an internal temperature of 361°C to carry out the methane production reaction 5. The ion intensity after 55 minutes is shown in Table 1 as "Reaction 5 (361°C)" intensity.

(Methane production reaction 6)

Following the methane production reaction 5, the temperature of the electric tubular furnace was increased at a rate of 5°C/min, and the catalyst i was maintained at an internal temperature of 41 1°C to carry out the methane production reaction 6. The ion intensity after 58 minutes is shown in Table 1 as "Reaction 6 (411°C)" intensity.

Fig. 1 shows the mass chromatograms of the ions measured by a mass spectrometer in the methane production reactions 1 to 6.

[Table 1]

| m/z | 2 | 15 | 16 | 18 | 28 | 44 |
|---|---|---|---|---|---|---|
| Baseline | 3.6 | 0.15 | 0.28 | 1.9 | 0.76 | 0.43 |
| Raw material gas | 3.7 | 2.40 | 9.8 | 2.3 | 0.97 | 4.2 |
| Reaction 1 (158°C) | 3.8 | 2.30 | 10 | 2.5 | 0.97 | 4.3 |
| Reaction 2 (209°C) | 4.3 | 2.20 | 10 | 2.5 | 0.98 | 4.3 |
| Reaction 3 (260°C) | 6.8 | 2.20 | 10 | 2.5 | 1.1 | 4.3 |
| Reaction 4 (311°C) | 16 | 2.20 | 9.6 | 2.6 | 1.3 | 4.1 |
| Reaction 5 (361°C) | 66 | 2.00 | 8.5 | 3.2 | 2.6 | 3.7 |
| Reaction 6 (411°C) | 200 | 1.60 | 3.6 | 5.8 | 6.1 | 2.5 |
| * The unit of each ion intensity is $\times 10^{-12A}$ (ampere). | | | | | | |

(Calculation of carbon dioxide conversion rate)

The carbon dioxide conversion rate was calculated using a formula (3) below from the ion intensity value at each stage of m/z=44 recorded by the mass spectrometer.

Carbon dioxide conversion rate (%) = ((1 - [(<reaction ◦ ((ΔΔΔ °C)> - <baseline>)] / (<raw material gas> - <baseline>)]))x100 (3) (3)

In the reaction ◦ (ΔΔΔ °C), the o is filled with a number from 1 to 6, and the ΔΔΔ is filled with its corresponding internal temperature number.

(Calculation of ammonia conversion rate)

The ammonia conversion rate was calculated using a formula (4) below from the ion intensity value at each stage of m/z= 16 recorded by the mass spectrometer.

Ammonia conversion rate (%) = ((1 - [(<reaction ◦ (ΔΔΔ °C)> - <baseline>)] / (<raw material gas> - <baseline>)]))×100 (4) (4)

In the reaction o temperature (ΔΔΔ °C), the ◦ is filled with a number from 1 to 6, and the ΔΔΔ is filled with its corresponding internal temperature number.

(Calculation of methane production rate)

From the methane production reaction formula shown in a formula (III) below, the methane production rate was calculated using a formula (5) below assuming the selectivity to be 100%.

$$CO_2+4H_2 \rightarrow CH_4+2H_2O+164.7kJ/mol \quad (III)$$

$$\text{Methane production rate (mmol/min)=carbon dioxide gas flow rate (cm}^3\text{ /min)} \times \text{carbon dioxide conversion rate/100/22.4} \quad (5)$$

(Calculation of hydrogen generation rate)

The hydrogen production rate was calculated from the hydrogen production reaction formula shown in a formula (II) below and the methane production reaction formula shown in a formula (III) below using a formula (6) below assuming the selectivity to be 100%.

$$NH_3 \rightarrow 1.5H_2+0.5N_2 - 45.9kJ/mol \quad (II)$$

$$\text{Hydrogen production rate (mmol/min) = ammonia gas flow rate (cm}^3\text{/min)} \times \text{ammonia conversion rate/100/22.4} \times 1.5 \text{ - methane production rate} \times 4 \quad (6)$$

The calculated carbon dioxide conversion rate, the ammonia conversion rate, the methane production rate, and the hydrogen production rate are shown in Table 2. When the calculated value was negative, it was stated as 0. The production of methane and hydrogen is also evident from the change in ion intensity at m/z = 15 and 2. Although the ion intensity of m/z = 15 does not simply increase due to overlap of fragments derived from ammonia, for example, from reaction 5 (361°C) to reaction 6 (411°C), the ion intensity of m/z= 16, which represents ammonia, decreases by 0.42 times from $8.5\times10^{-12}$ A to $3.6\times10^{-12}$ A, whereas the ion intensity of m/z=15, which represents fragments derived from methane and ammonia, decreases by only 0.80 times from $2.0\times10^{-12}$ A to $1.6\times10^{-12}$ A.

[Table 2]

| | Carbon dioxide conversion rate (%) | Ammonia conversion rate (%) | Methane production rate (mmol/min) | Hydrogen production rate (mmol/min) |
|---|---|---|---|---|
| Reaction 1 (158°C) | 0 | 0 | 0 | 0 |
| Reaction 2 (209°C) | 0 | 0 | 0 | 0 |
| Reaction 3 (260°C) | 0 | 0 | 0 | 0 |
| Reaction 4 (311°C) | 3 | 2 | 0.0012 | 0 |
| Reaction 5 (361°C) | 13 | 14 | 0.0052 | 0.0091 |
| Reaction 6 (411°C) | 45 | 65 | 0.018 | 0.021 |

Using the formulas (II) and (III), the calculated values of the reaction heat required to produce 1 mol of methane are shown in Table 3, in which the molar ratio of ammonia to carbon dioxide in the raw material gas is changed.

[Table 3]

| | Molar ratio of ammonia to carbon dioxide in the raw material gas | Reaction heat (kJ/mol) |
|---|---|---|
| Test example 1 | 3.6 | 0.18 |
| Test example 2 | 3.0 | 27 |
| Test example 3 | 5.4 | -83 |
| Test example 4 | 2.7 | 42 |
| Test example 5 | 6.0 | -110 |

As is clear from Tables 2 and 3, the method for producing methane according to the present disclosure is able to

produce methane with excellent reaction efficiency from a raw material gas containing ammonia and carbon dioxide in the presence of a catalyst containing a carrier and a transition metal. In particular, Test Examples 1 to 3, in which the molar ratio of ammonia to carbon dioxide in the raw material gas was 3.0 or more and 5.4 or less, resulted in an exothermic reaction with a reaction heat of 30 kJ/mol or less, or an endothermic reaction with a reaction heat of 100 kJ/mol or less, and were able to produce methane with superior reaction efficiency.

[Production Example 2]

<Preparation of catalyst precursor>

50 parts by mass of rutile crystalline titanium dioxide (STR-60R, manufactured by Sakai Chemical Industry Co., Ltd., 100% rutile crystalline titanium dioxide) and 50 parts by mass of α-alumina (AES-12, manufactured by Sumitomo Chemical Co., Ltd.) were mixed, and then 12.8 parts by mass of titanium dioxide sol (CSB, manufactured by Sakai Chemical Industry Co., Ltd., titanium dioxide content in titanium dioxide sol: 39% by mass, titanium dioxide is 100% anatase crystalline titanium dioxide) was diluted with pure water per 100 parts by mass of this mixture, and the mixture was kneaded. This kneaded material was extruded into a cylindrical shape with a diameter of 1.5 mm, dried, and then crushed into pieces with a length of about 2 to 4 mm. The resulting molded body was calcined in air at 650 to 680 °C for 3 hours to obtain a carrier ii of a mixture of titanium dioxide and α-alumina. This carrier ii was impregnated with an aqueous solution of a commercially available ruthenium chloride hydrate, dried, and then calcined in air at 250°C for 2 hours, thereby obtaining a catalyst precursor ii-1 in which ruthenium oxide was supported on the carrier at a loading of 4%.

<Preparation of catalyst>

A catalyst precursor ii-1 was pulverized and sieved to 75 to 150 μm, and then 2.8 g was weighed out and filled into a SUS reaction tube with an inner diameter of 7.5 mm and equipped with a sheath tube with an outer diameter of 3 mm for measuring the internal temperature. Using an electric tubular furnace, the internal temperature was raised to 500 °C under the flow of helium of 110 $cm^3$ (0 °C, 0.1013 MPa (absolute))/min, and maintained at the same temperature for 1 hour. The internal temperature was then lowered to 400 °C and maintained for 1 hour under the flow of hydrogen of 5 $cm^3$ (0 °C, 0.1013 MPa (absolute))/min and the flow of helium of 110 $cm^3$ (0 °C, 0.1013 MPa (absolute))/min to obtain a catalyst ii. The catalyst ii was cooled to an internal temperature of 150 to 160 °C under the flow of helium of 100 $cm^3$ (0 °C, 0.1013 MPa (absolute))/min, and used as is in the methane production reaction.

<Methane production reaction>

(Gas analysis by in-line gas chromatography)

The post-reaction gas discharged from the SUS reaction tube was introduced into an in-line gas chromatography (GL Sciences Inc., Agilent 990 Micro GC) to analyze the reaction gas. The analysis conditions are as follows:

Carrier gas: Helium
Injection temperature: 100 °C
Column used: COX UM 1m × 0.8 mm ID ST
Column temperature: 100 °C
Pressure: 170 kPa

(Creating a calibration curve)

To calculate the composition ratio in the exhaust gas, a calibration curve was created according to the following procedures.

Using a gas flow meter, a raw material gas comprising a mixture in a range that a methane (16%) + nitrogen (84%) mixed gas at a rate of 0 to 75 $cm^3$ (0 °C, 0.1013 MPa (absolute))/min, a carbon dioxide gas at a rate of 0 to 15 $cm^3$ (0 °C, 0.1013 MPa (absolute))/min, a helium gas at a rate of 11 to 95 $cm^3$ (0 °C, 0.1013 MPa (absolute))/min, and a hydrogen gas at a rate of 0 to 53 $cm^3$ (0 °C, 0.1013 MPa (absolute))/min, was made to flow through an SUS reaction tube not packed with a catalyst and gas analysis was performed. The peak position of each component was confirmed from the obtained GC chart, and the area values between the following R.T.s (retention times) were recorded to prepare a calibration curve of GC area values versus gas volume percentage: hydrogen: 14.1-21.1 s, nitrogen: 30.0-38.5 s, methane: 73.3-100.0 s, carbon dioxide: 146.4-183.1 s.

(Methane production reaction 7)

The catalyst ii packed in the SUS reaction tube was heated to an internal temperature of 400 °C., using an electric tubular furnace while flowing helium gas at 115 $cm^3$ (0 °C., 0.1013 MPa (absolute))/min. After the temperature reached 400 °C, using a gas flow meter, a raw material gas comprising a mixture in a range that an ammonia gas at 40 $cm^3$ (0 °C, 0.1013 MPa (absolute))/min, a carbon dioxide gas at 15 $cm^3$ (0 °C, 0.1013 MPa (absolute))/min, and a helium gas at 60 $cm^3$ (0 °C, 0.1013 MPa (absolute))/min was made to flow through the SUS reaction tube (the molar ratio of ammonia to carbon dioxide in the raw material gas was 2.7). After making the gas to flow for 1 hour, the exhaust gas was analyzed and the resulting GC area values were recorded.

(Methane production reaction 8)

Following the methane production reaction 7, a raw material gas comprising a mixture in a range that an ammonia gas at 40 $cm^3$ (0 °C, 0.1013 MPa (absolute))/min, a carbon dioxide gas at a rate of 15 $cm^3$ (0 °C, 0.1013 MPa (absolute))/min, a hydrogen gas at 7.5 $cm^3$ (0 °C, 0.1013 MPa (absolute))/min, and a helium gas at a rate of 52.5 $cm^3$ (0 °C, 0.1013 MPa (absolute))/min), was made to flow through the SUS reaction tube (the molar ratio of ammonia to carbon dioxide in the raw material gas was 2.7). After making the gas to flow for 1 hour, the exhaust gas was analyzed and the resulting GC area values were recorded.

(Methane production reaction 9)

Following the methane production reaction 8, a raw material gas comprising a mixture in a range that an ammonia gas at 40 $cm^3$ (0 °C, 0.1013 MPa (absolute))/min, a carbon dioxide gas at a rate of 15 $cm^3$ (0 °C, 0.1013 MPa (absolute))/min, a hydrogen gas at 15 $cm^3$ (0 °C, 0.1013 MPa (absolute))/min, and a helium gas at a rate of 45 $cm^3$ (0 °C, 0.1013 MPa (absolute))/min), was made to flow through the SUS reaction tube (the molar ratio of ammonia to carbon dioxide in the raw material gas was 2.7). After making the gas to flow for 1 hour, the exhaust gas was analyzed and the resulting GC area values were recorded.

(Methane production reaction 10)

Following the methane production reaction 9, a raw material gas comprising a mixture in a range that an ammonia gas at 40 $cm^3$ (0 °C, 0.1013 MPa (absolute))/min, a carbon dioxide gas at a rate of 15 $cm^3$ (0 °C, 0.1013 MPa (absolute))/min, a hydrogen gas at 30 $cm^3$ (0 °C, 0.1013 MPa (absolute))/min, and a helium gas at a rate of 30 $cm^3$ (0 °C, 0.1013 MPa (absolute))/min), was made to flow through the SUS reaction tube (the molar ratio of ammonia to carbon dioxide in the raw material gas was 2.7). After making the gas to flow for 1 hour, the exhaust gas was analyzed and the resulting GC area values were recorded.

Table 4 shows the gas flow rates and the nitrogen and methane production rates (%) in the methane production reactions 7 to 10. In Table 4, $NH_3$ is ammonia, $CO_2$ is carbon dioxide, $H_2$ is hydrogen, He is helium, $N_2$ is nitrogen, and $CH_4$ is methane.

The production rates shown in Table 4 were calculated using the following formula.

$CH_4$ (%) = $CH_4$ volume % determined from the calibration curve / $CH_4$ theoretical volume % when all of the introduced $CO_2$ is converted to $CH_4$ × 100

$N_2$ (%) = $N_2$ volume % determined from the calibration curve / $N_2$ theoretical volume % when all of the introduced $NH_3$ is decomposed into $N_2$ and $H_2$ × 100

[Table 4]

| | Flow rate ($cm^3$/ min) | | | | Production rate (%) | |
|---|---|---|---|---|---|---|
| | $NH_3$ | $CO_2$ | $H_2$ | He | $N_2$ | $CH_4$ |
| Reaction 7 | 40 | 15 | 0 | 60 | 43 | 34 |
| Reaction 8 | 40 | 15 | 7.5 | 52.5 | 45 | 53 |
| Reaction 9 | 40 | 15 | 15 | 45 | 50 | 77 |

(continued)

| | Flow rate ($cm^3$/ min) | | | | Production rate (%) | |
|---|---|---|---|---|---|---|
| | $NH_3$ | $CO_2$ | $H_2$ | He | $N_2$ | $CH_4$ |
| Reaction 10 | 40 | 15 | 30 | 30 | 54 | >99 |

As is clear from Table 4, when the raw material gas contains hydrogen in addition to ammonia and carbon dioxide, methane can be produced more efficiently. The improvement in methane production efficiency due to the raw material gas containing hydrogen is particularly effective when the molar ratio of ammonia to carbon dioxide in the raw material gas is less than 3.0. In this reaction, methane and hydrogen can be produced from a gas containing ammonia and carbon dioxide in the presence of a catalyst containing a carrier and a transition metal. When it is desired to produce mainly methane, the efficiency of methane production can be increased by recycling the produced hydrogen into the raw material gas.

[Production Example 3]

<Methane production reaction from ammonium carbonate ($(NH_4)_2CO_3$ )>

(Preparation of catalyst)

The catalyst was prepared as described in Production Example 2. The amount of catalyst used in the reaction was 1.6 g, and the reaction was carried out in a quartz glass reaction tube with an inner diameter of 12 mm equipped with a sheath tube with an outer diameter of 3 mm.

(Preparation of $(NH_4)_2CO_3$ aqueous solution)

The $(NH_4)_2CO_3$ aqueous solution used in the reaction was prepared using commercially available $(NH_4)_2CO_3$.

(Methane production reaction 11)

The catalyst packed in the quartz glass reaction tube was heated to an internal temperature of 400 °C using an electric tubular furnace with helium gas fed at 115 $cm^3$ (0 °C, 0.1013 MPa (absolute))/min. After the temperature reached 400 °C, a 16.7 mass% $(NH_4)_2CO_3$ aqueous solution was fed to the upper part of the reaction tube at a rate of 1 mL/min, and the aqueous solution was impregnated into wool placed above. Using a gas flow meter, hydrogen gas was made to flow through the upper part of the reaction tube at a rate of 15 $cm^3$ (0 °C., 0.1013 MPa (absolute))/min. The vaporized $(NH_4)_2CO_3$ component and hydrogen gas were mixed and introduced at the top of the reaction tube and brought into contact with a catalyst layer (the molar ratio of ammonia to carbon dioxide in the raw material gas was 2.0). After 1 hour of flowing, an exhaust gas was analyzed and the obtained GC area values were recorded.

A methane peak was confirmed on the obtained GC chart, and the methane production rate was confirmed by the same evaluation method as in Production Example 2. The methane production rate was confirmed to be 28.5%. Therefore, it is found that methane can be produced even when the raw material gas is derived from the gas obtained by thermal decomposition of $(NH_4)_2CO_3$.

## Claims

1. A method for producing methane comprising: producing methane from a raw material gas comprising ammonia and carbon dioxide in the presence of a catalyst comprising a carrier and a transition metal.

2. The method for producing methane according to claim 1, wherein the molar ratio of ammonia to carbon dioxide in the raw material gas is 3.0 or more and 5.4 or less.

3. The method for producing methane according to claim 1 or 2, wherein the raw material gas further comprises hydrogen.

4. The method for producing methane according to claim 2, wherein hydrogen is produced from the raw material gas simultaneously with the production of methane.

5. The method for producing methane according to claim 3, wherein hydrogen contained in the raw material gas is obtained by recovering hydrogen resulting from the production of methane, and recycling the recovered hydrogen into the raw material gas.

6. The method for producing methane according to claim 4, wherein hydrogen contained in the raw material gas is obtained by recycling a stream containing hydrogen resulting from the production of methane.

7. The method for producing methane according to claim 1 or 2, wherein a reaction temperature in the production of methane is 300°C or higher and 550°C or lower.

8. The method for producing methane according to claim 1 or 2, wherein a reaction pressure in the production of methane is 0 MPa-G or more and 0.9 MPa-G or less.

9. The method for producing methane according to claim 1 or 2, wherein the carrier is at least one carrier selected from the group consisting of titania, alumina, silicon carbide, carbon, zirconia, silica, cerium oxide, and niobium oxide.

10. The method for producing methane according to claim 9, wherein the carrier has pores with an average pore diameter of 2 nm or more and 100 nm or less.

11. The method for producing methane according to claim 2, wherein the transition metal is ruthenium.

12. The method for producing methane according to claim 3, wherein the transition metal is ruthenium.

13. The method for producing methane according to claim 9, wherein the transition metal is ruthenium.

14. The method for producing methane according to claim 1 or 2, wherein the production of methane is carried out using a reactor, and the reactor is at least one selected from the group consisting of an external heat exchange type fixed bed reactor, an adiabatic fixed bed reactor, a fluidized bed reactor, a simulated moving bed reactor, a riser type fluidized bed reactor, and a radial flow type fixed bed reactor.

15. The method for producing methane according to claim 1, wherein the raw material gas comprises a gas obtained by thermal decomposition of ammonium carbonate.

16. The method for producing methane according to claim 15, wherein the ammonium carbonate is obtained by bringing an aqueous solution comprising ammonia into contact with a gas comprising carbon dioxide.

17. The method for producing methane according to claim 16, wherein the gas comprising carbon dioxide is air.

18. The method for producing methane according to claim 16, wherein the gas comprising carbon dioxide is an exhaust gas from a power plant, a factory, a steel mill, a chemical plant, a waste treatment facility, or a biomass facility.

Fig.1

m/z=2(A)
m/z=15(A)
m/z=16(A)
m/z=18(A)
m/z=28(A)
m/z=44(A)
ion intensity(m/z=2)
ion intensity(m/z=15, 16, 18, 28, 44)
Time(s)
m/z=16
m/z=2
m/z=28
m/z=44
m/z=18
m/z=15
2.5E-10
2.0E-10
1.5E-10
1.0E-10
5.0E-11
0.0E+00
1.6E-11
1.4E-11
1.2E-11
1.0E-11
6.0E-12
4.0E-12
2.0E-12
0
5000
10000
15000
20000
25000
30000

**INTERNATIONAL SEARCH REPORT**

International application No. **PCT/JP2024/034286**

**A. CLASSIFICATION OF SUBJECT MATTER**

***C07C 1/02***(2006.01)i; ***B01J 23/58***(2006.01)i; ***C01B 3/04***(2006.01)i; ***C07B 61/00***(2006.01)i; ***C07C 1/12***(2006.01)i; ***C07C 9/04***(2006.01)i

FI: C07C1/02; C07C9/04; B01J23/58 M; C07B61/00 300; B01J23/58 Z; C07C1/12; C01B3/04 B

According to International Patent Classification (IPC) or to both national classification and IPC

**B. FIELDS SEARCHED**

Minimum documentation searched (classification system followed by classification symbols)

C07C1/02; B01J23/58; C01B3/04; C07B61/00; C07C1/12; C07C9/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2014-5533 A (JFE STEEL CORPORATION) 16 January 2014 (2014-01-16) claims, paragraphs [0005], [0013], [0020]-[0022], examples | 1-18 |
| X | UDDIN, A. M. et al. Catalysis Today. 2017, 291, 24-28 abstract, 2.1-3.3, fig. 1-2 | 1-18 |
| X | 今村壮甫　他, Ｒｕ系複合触媒を用いたＣＯ２からのアンモニアメタネーションにおける反応条件の影響, 日本エネルギー学会大会講演要旨集　第32回日本エネルギー学会大会, 01 August 2023, 10-11, (IMAMURA, Sosuke et al., Effects of Reaction Conditions on Ammonia Methanation from CO2 with Ruthenium-based Hybrid Catalysts, Proceedings of the Annual Conference of the Japan Institute of Energy: The 32nd Annual Conference of the Japan Society of Energy) entire text, particularly, Introduction, Experiment, Results and discussion | 1-18 |
| A | SAIMA, H. et al. Journal of Chemical Engineering of Japan. 14 September 2023, vol. 56, no. 1, 2248176 entire text, all drawings | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2024** | **26 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/034286**

**C. DOCUMENTS CONSIDERED TO BE RELEVANT**

| Category* | Citation of document, with indication, where appropriate, of the relevant passages | Relevant to claim No. |
|---|---|---|
| A | 齐間等　他, 沈殿鉄系ハイブリッド触媒を用いたアンモニアメタネーション, 日本エネルギー学会大会講演要旨集　第３１会日本エネルギー学会大会, 90-91, (SAIMA, Hitoshi et al., Ammonia-Methanation with a Hybrid Catalyst of Precipitated Iron, Proceedings of the Annual Conference of the Japan Institute of Energy: The 31st Annual Conference of the Japan Institute of Energy) entire text, all drawings | 1-18 |
| A | 齐間等　他, アンモニアによる炭酸ガスからのメタン合成, 日本エネルギー学会大会講演要旨集　第３０回日本エネルギー学会大会, 2021, 116-117, (SAIMA, Hitoshi et al., Methanation of Carbon Dioxide with Ammonia), non-official translation (Proceedings of the Annual Conference of the Japan Institute of Energy: The 30th Annual Conference of the Japan Institute of Energy) entire text, all drawings | 1-18 |
| A | CN 112340843 A (BEIJING UNIVERSITY OF TECHNOLOGY) 09 February 2021 (2021-02-09) entire text, all drawings | 1-18 |
| A | WO 2022/224993 A1 (OSAKA GAS CO., LTD.) 27 October 2022 (2022-10-27) entire text, all drawings | 1-18 |

Form PCT/ISA/210 (second sheet) (July 2022)

**INTERNATIONAL SEARCH REPORT**
**Information on patent family members**

International application No.
**PCT/JP2024/034286**

| Patent document cited in search report | Publication date (day/month/year) | Patent family member(s) | Publication date (day/month/year) |
|---|---|---|---|
| JP 2014-5533 A | 16 January 2014 | (Family: none) | |
| CN 112340843 A | 09 February 2021 | (Family: none) | |
| WO 2022/224993 A1 | 27 October 2022 | US 2024/0181435 A1<br>entire text, all drawings<br>EP 4327937 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023180411 A
- JP 2024159324 A
- JP 2010194519 A
- WO 2022224993 A